# EUROPEAN PATENT APPLICATION

(11) **EP 4 631 873 A1**
(43) Date of publication of application: **15.10.2025**
(21) Application number: 24836856.5
(22) Date of filing: 04.01.2024

(54) **LIQUID DISPENSER WITH A TIP OPENING MECHANISM OF THE TYPE WITHOUT PRESERVATIVES FOR BLOW-FILL-SEAL CONTAINERS**

(71) Applicant: Gamboa Burgos, Alejandro, Lima 05 (PE)
(72) Inventor: Gamboa Burgos, Alejandro, Lima 05 (PE)
(74) Representative: Pons IP
(86) International application number: PCT/PE2024/050001
(87) International publication number: WO 2025/147199

(57) **Abstract**

The present invention relates to a liquid dispenser with an opening by tip mechanism, of the type without preservatives for bottles of blowing, filling and sealing, with a dual-function membrane for the air-and-liquid passage control, with a pierceable silicone seal that ensures sterilization at all times by being in storage position and when opening the bottle.

## Description

### TECHNICAL FIELD

The present invention falls within the technical field of ophthalmic product dispensers, specifically a multidose drop dispensing device with an opening by tip mechanism (spike system), suitable for solutions without preservatives for plastic bottles made by blowing, filling, and sealing (Blow-fill-seal), is disclosed.

### STATE OF THE ART

In the state of the art, there are some technologies related to multidose droppers without preservatives, such as the US11116694B2 patent document, published on September 14, 2021, for «Device for dispensing liquid from a sterile packaging bottle», which relates to a device for dispensing aqueous liquids through an interface membrane made partially hydrophilic and partially hydrophobic, made in such a way that when in operation, during each operation of dispensing a metered amount of liquid, the streams of air and liquid flow alternately in a capillary channel downstream from the membrane. Said interface membrane is made of a filtering material which includes biocidal metal cations in the body thereof; likewise, it includes a porous insert, permeable both to liquid and to air, which is arranged upstream from the membrane in the fluid path. However, this background lacks a tip mechanism with a silicone seal that tightens to the spout of the bottle by being perforated, keeping all the components sterile during the process.

Likewise, with respect to dual-function membranes, that is, hydrophilic and hydrophobic, the US6565748B1 patent document, published on May 20, 2003, for «Cationically charge-modified membranes», is known, which discloses a charge-modified polymer membrane, said membrane comprising a hydrophobic polymer, said membrane being rendered hydrophilic by charge modification, said charge modification resulting from contacting said membrane with at least one polymeric wetting agent and at least one cationic charge modifying agent. However, this background simply discloses a dual-function membrane and does not include a body and a circular platform to fix the membrane; it does not reveal either a tip mechanism communicated with the camera created between the body and the circular platform.

Additionally, the background of the state of the art having an opening by tip mechanism for drop bottles loses sterility by breaking the bottle.

Further, conventional multidose bottles without preservatives have the expiration time limited to periods below one and a half year (the US Food and Drug Administration (FDA) does not deliver further time for this type of containers), since liquids are in contact with a number of elements that could be contaminant.

### DESCRIPTION OF THE INVENTION

With the aim of solving the aforementioned problems from the state of the art, the present invention was developed, which relates to a liquid-dispensing device, which comprises a bottle with a lower, an intermediate and an upper portion, where the intermediate portion has a diameter smaller than a lower portion of the bottle, the upper portion of the bottle is threaded and has a pierceable spout; a main support body, where said main support body includes: a threaded inner surface, an inner annular protuberance arranged above the threaded inner surface, wherein a radial extension of a silicone seal fits in, the silicone seal (which is pierceable) having a tip-shaped cavity and being located inside the main support body, the inner upper part of the main support body includes a tip of the main support body with an inner channel, whereas the outer upper part of the main support body has a first grooved platform, wherein a membrane (dual-function membrane: hydrophilic and hydrophobic) is supported, a threaded outer surface arranged below the first grooved platform; the membrane is arranged over the first grooved platform and below a second grooved platform, the second grooved platform having a through hole and a vertically extending protuberance;
wherein the first grooved platform and the second grooved platform increase the contact surface of fluids with the membrane,
wherein the membrane es partially hydrophilic and partially hydrophobic;
wherein a lid with a lid thread is suitable to be threaded on the threaded outer surface of the main support body.

In a use position, the spout of the bottle is in direct contact with the silicone seal, the tip of the main support body is arranged inside the bottle and the silicone seal is perforated.

Whereas, in a storage position, the silicone seal, the membrane and the channels created around the dual-function membrane are kept sterile and without perforating the silicone seal, preferably, the tip of the main support body is in permanent contact with the silicone seal, pressing without perforating.

The present invention manages to integrate a set of relevant technical characteristics in a single device, providing the following technical effects:
- Durability in storage state is increased, with up to three-year expiration, compared to 1 to 1,5 years of the conventional multidose dispensing bottles without preservatives from the state of the art.
- The tip of the main body and the silicone seal, which preferably contain silver ions as bactericide, all the time that are in storage position, are sterile. The tip is never exposed to the environment, there not being an opening mechanism by breakdown with tip, with solution without preservatives, in the state of the art.
- In storage position, the tip of the bottle is in permanent contact with the soft silicone seal containing silver ions as bactericide, always keeping the external part of the tip of the bottle under sterile condition.
- When perforating the silicone seal and the tip of the bottle, the sterility of the whole drop dispenser is kept, without allowing the air entering through any site. Both perforations are performed in a single operation.
- By being the channel of the tip of the main support body located in a side area, it is allowed that it may be made with the perforated tip centered, and of hard plastic or other materials, such as stainless steel.
- The dual-function membrane, that is, partially hydrophobic and partially hydrophilic, during the drop dispensing, allows for fluids to be guided through the return path thereof, allowing for the remaining liquid not expelled (returning first) to deviate from a direct axial trajectory and point to the hydrophilic part of the membrane, thus the air flow drawn into the bottle has free access to the hydrophobic material of the membrane in the central portion thereof.
- Once the main support body is threaded up to the bottom of the bottle, said main body cannot longer be removed and is kept permanently in use position; likewise, the silicone seal will be kept pressing the tip of the bottle, sealing it; at the same time, the safety ring comes off over the intermediate portion of the bottle as a visual indicator that the bottle was perforated and is in use position.
- The channels of the first and second grooved platforms allow for the return fluids to cover a greater surface of the dual-function membrane.

### BRIEF DESCRIPTION OF THE FIGURES

The following figures illustrate some embodiments of the present invention, said figures or the description thereof not being intended to limit in any way the scope of protection defined by the claims.
**Figure 1****:** Internal view of the proposed invention in storage position.
**Figure 2****:** Internal view of the proposed invention in use position.
**Figure 3****:** View of the individual components of the proposed invention.
**Figure 4****:** View of the sealing pieces before the application of ultrasound process.
**Figure 5****:** View of the sealing pieces after the application of ultrasound process.

Legend of the figures:
10: Bottle
11: Lower portion
12: Intermediate portion
13: Upper portion
14: Spout of the bottle
15: Fin of the intermediate portion
20: Main support body
21: Inner annular protuberance of sealing
21a: Inner annular protuberance of sealing before ultrasound
21b: Inner annular protuberance of sealing after ultrasound
22: Threaded inner surface
23: Threaded outer surface
24: Tip of the main support body
25: Inner channel of the tip
26: First grooved platform
27: Tooth of the main support body
28: Outer annular protuberance of sealing
28a: Outer annular protuberance of sealing before ultrasound
28b: Outer annular protuberance of sealing after ultrasound
30: Silicone seal
31: Radial extension of the silicone seal
32: Tip-shaped cavity of the silicone seal
33: Free space
40: Membrane
50: Second grooved platform
51: Vertically extending protuberance of the second grooved platform
60: Lid
61: Lid tooth
62: Lid thread
70: Safety ring
71: Tooth of the safety ring

### PREFERRED EMBODIMENT OF THE INVENTION (DETAILED DESCRIPTION OF THE INVENTION)

In a preferred embodiment, the silicone seal (30) is soft (5-20 Shore A hardness).

In another preferred embodiment, the tip-shaped cavity of the silicone seal (32) has a diameter smaller than the tip of the main support body, so that when the silicone seal is coupled and introduced, both elements are tightened under pressure. Likewise, this silicone seal has some inner walls that fit with the external form of the spout of the bottle, so that a seal is created under pressure between the spout of the bottle and the silicone seal, in the use position.

In a preferred and detailed embodiment, the tip of the main support body (24) is formed by a cylindrical portion followed by a sharp termination, wherein a side section of the sharp termination has a fluid conduit channel, so that the tip doing the perforation does not lose the edge thereof, and perforation is eased to the user by employing less strength, even being able to make said tip of a material less hard than steel, such as plastic. The fluid conduit channel located in the side area of the tip is connected with an inner channel of the tip carrying the liquid to the membrane.

In the use position of the present invention, the arranged liquid conduit channel (side channel) remains inside the bottle. Likewise, the tip of the main support body (24) may be of plastic or stainless steel.

Likewise, in a preferred embodiment, the main support body (20) has, in the lower part (part nearest to the bottle), a safety ring (70) (annular protuberance) with teeth (71), which serve as a stop to the rotation of the safety ring. Likewise, the lid (60) has a lid thread (62) suitable to be threaded on the threaded outer surface of the main support body (23).

In another preferred embodiment, the bottle may be made of PET-G or polyethylene, polypropylene, among other similar plastics; and it may be made by blowing, filling, and sealing (Blow-fill-seal) technologies.

In another preferred embodiment, the bonding between the first grooved platform, the dual-function membrane and the second grooved platform is an ultrasonic bonding. Likewise, the silicone seal is also coupled by casting the inner annular protuberance of sealing with ultrasound (21).

In a preferred embodiment, the intermediate portion of the bottle (12) comprises at least two fins (15), preferably between 2 and 4 fins, where the fins (15) are arranged with a slope, that is, as the main support body is being introduced, this is fitted under pressure into the bottle.

In another preferred embodiment, the second grooved platform (50) has a central port, with sloping walls, that is, the port would have a frusto-conical form with a modifiable diameter to adjust the size of the drop as needed.

In another preferred embodiment, the liquid-dispensing device is sterilized by gamma rays or ethylene oxide.

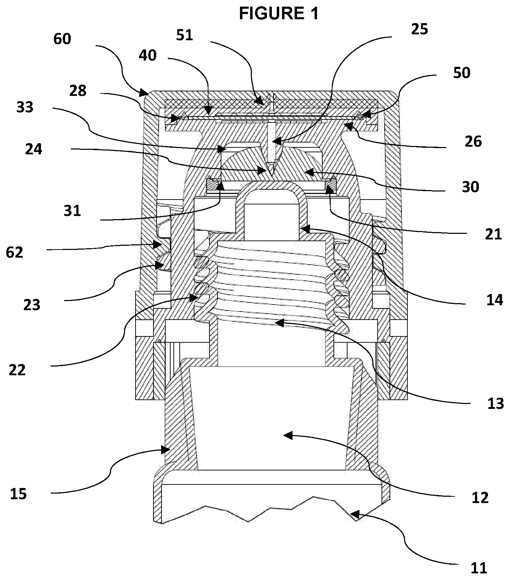

In Figure 1, the present invention is shown in storage position, in which position the tip of the main support body (24) is substantially close or in contact with the silicone seal (30), with a free space (33) between said silicone seal and the inner upper portion of the main support body.

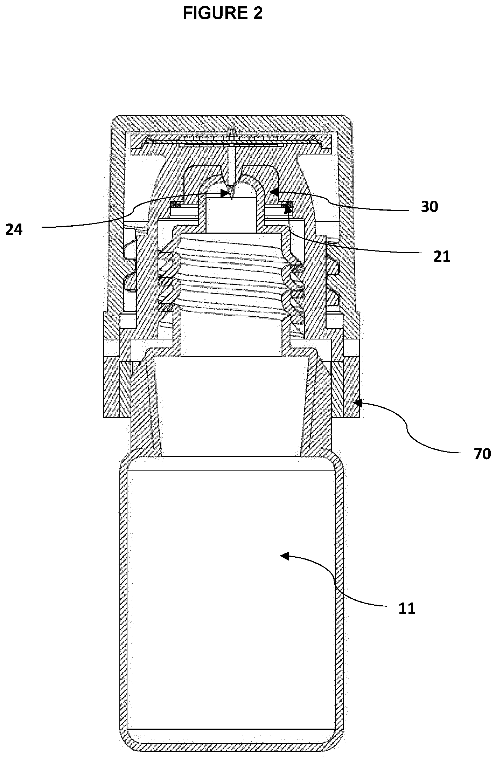

As can be seen in Figure 2, by performing the threading of the main support body (20) over the upper portion (13) of the bottle, the main support body (20) falls and makes holes in the silicone seal (30), so that the silicone seal (30) is displaced and adapted by pressure over the free space (33) and tightened over the spout of the bottle (14).

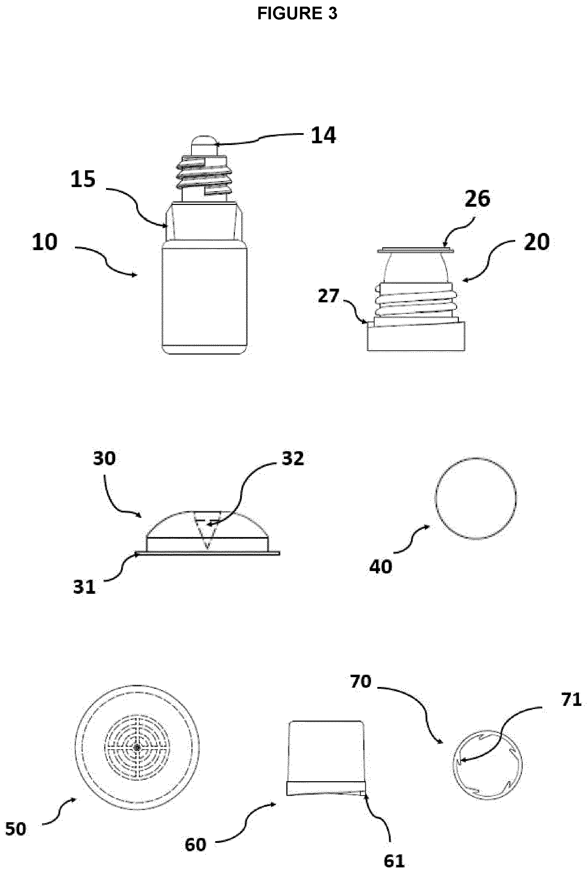

In Figure 3, the pieces of the proposed invention are seen, wherein inside the main support body (20) the silicone seal (30) is located, wherein the sealing silicone is in the shape of a bulged body with a radial extension in the lower part thereof, which functions as a stop by being placed inside the sealing body, wherein, by being the silicone seal (30) coupled over the main support body, a sterilized and free space (33) is kept between the upper and inner walls of the main support body and the silicone seal (30), that is, the upper part of the sealing silicone does not initially contact the upper wall of the main support body, it only contacts the tip of the main support body. In the storage position, the tip of the main support body (24) lies over the tip-shaped cavity of the silicone seal (32). In this Figure 3, the safety ring (70) can also be visualized, which is coupled by ultrasound with the lid of the bottle, this safety ring being suitable to fit over the fins (15) of the bottle, so that when the bottle is perforated for the first time by applying the rotation of pieces, said safety ring (70) is detached from the lid of the bottle, indicating that the bottle is in use position and no longer in storage position. The safety ring (70) includes some backpressure teeth (71) that stop the rotation of said ring by contacting the fins (15) of the bottle, so that the detachment from the safety ring is eased.

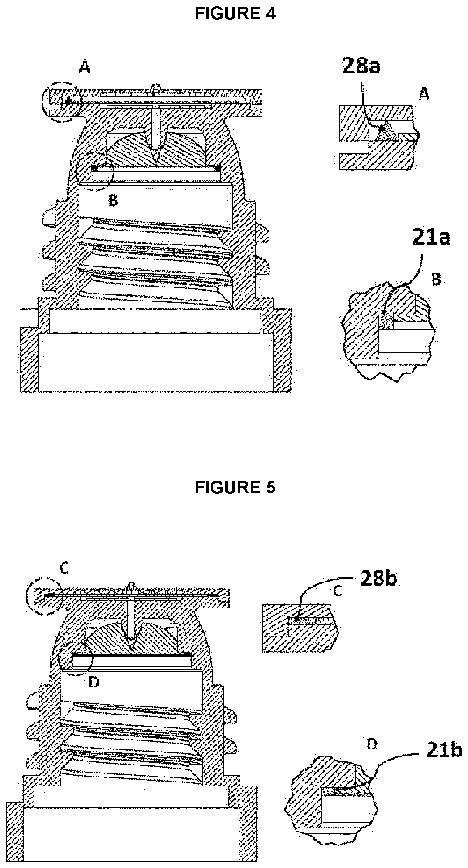

In Figures 4 and 5, the element bonding process by ultrasound of the proposed invention can be seen, wherein the outer annular protuberance of sealing (28) initially has a pointed annular shape, that is, seen from a sectional cut to the ring, it has a triangular shape, as seen in Figure 4; and the inner annular protuberance of sealing (21) initially has the shape of an annular notch, that is, protruding annularly to the inside of the main support body; both protuberances are made in the same plastic material of the main support body and, by applying ultrasound over said protuberances, these are casted and allow for the bonding with other elements; the inner annular protuberance of sealing allows for the fixation of the sealing silicone (3) over the main support body, remaining part of the casted material over the radial extension of the silicone seal (31); on the other hand, the outer annular protuberance of sealing (28) allows for the fixation of the second grooved platform (50) over the first grooved platform (26) of the main support body.

## Claims

1. A liquid-dispensing device **characterized by** comprising:
a bottle (10) with a lower portion (11), an intermediate portion (12) and an upper portion (13), where the upper portion (13) of the bottle is threaded and has a pierceable spout (14);
a main support body (20), where said main support body includes:
a threaded inner surface (22), an inner annular protuberance (21) arranged above the threaded inner surface (22), wherein a radial extension of a silicone seal (31) fits in, the silicone seal (30) having a tip-shaped cavity (32) and being located inside the main support body, the inner upper part of the main support body includes a tip of the main support body (24) with an inner channel (25), whereas the outer upper part of the main support body has a first grooved platform (26), wherein a membrane (40) is supported,
a threaded outer surface (23) arranged below the first grooved platform (26);
the membrane (40) is arranged over the first grooved platform (26) and below a second grooved platform (50), the second grooved platform (50) having a through hole and an extending protuberance (61);
wherein, by being the silicone seal (30) coupled over the main support body, a free space (33) is kept between the upper and inner walls of the main support body and the silicone seal (30);
wherein the first grooved platform (26) and the second grooved platform (50) increase the contact surface of fluids with the membrane (40),
wherein the membrane (40) es partially hydrophilic and partially hydrophobic;
wherein a lid (60) with a lid thread (62) is suitable to be threaded on the threaded outer surface (23) of the main support body.

2. The liquid-dispensing device according to claim 1, **characterized in that** the lower part of the lid (60) has some lid teeth (61) that serve as a stop against some teeth of the main support body (27) arranged over an outer lower annular protuberance of the main support body.

3. The liquid-dispensing device according to claim 1, **characterized in that** the intermediate portion of the bottle (12) comprises at least two fins (15), where the fins (15) are arranged with a slope, that is, as the main support body is being introduced, this is fitted under pressure into the bottle.

4. The liquid-dispensing device according to claim 1, **characterized in that** the tip-shaped cavity of the silicone seal (32) has a diameter smaller than the tip of the main support body (24), so that when the silicone seal (30) is coupled and introduced, both elements are tightened under pressure.

5. The liquid-dispensing device according to claim 1, **characterized in that** the tip of the main support body (24) is formed by a cylindrical portion followed by a sharp termination, wherein a side section of the sharp termination has a fluid conduit channel and is connected with an inner channel of the tip, the conduit channel being located in a side area contributes to avoid the edge in the perforation.

6. The liquid-dispensing device according to claim 1, **characterized in that** the tip of the main support body (24) is made of plastic or stainless steel.

7. The liquid-dispensing device according to claim 1, **characterized in that** the silicone seal (30) is soft.
